# EUROPEAN PATENT SPECIFICATION

(11) **EP 0 747 056 B1**
(45) Date of publication and mention of the grant of the patent: **15.12.1999**
(21) Application number: 96303874.0
(22) Date of filing: 30.05.1996
(51) Int. Cl.: A61K 31/445, A61K 31/40, A61K 31/55, A61K 31/535, A61K 31/565, A61K 38/29

(54) **Minimizing of bone loss with anti-estrogen combinations**
Verringerung von Knochenschwund durch Antiöstrogen-Kombinationen
Diminuer la perte osseuse par des combinaisons d'anti-estrogènes

(30) Priority: 06.06.1995 US 471111
(43) Date of publication of application: 11.12.1996
(73) Proprietor: ELI LILLY AND COMPANY, Indianapolis, Indiana 46285 (US)
(72) Inventor: Cullinan, George Joseph, Trafalgar, Indiana 46181 (US); Fontana, Steven Anthony, Wilmington, North Carolina 28409 (US); Fuchs-Young, Robin Sharon Lee, Trafalgar, Indiana 46181 (US); Glasebrook, Andrew Lawrence, Zionsville, Indiana 46077 (US)
(74) Representative: Tapping, Kenneth George

(56) References cited:
- DE-A- 4 329 344
- US-A- 4 895 715
- US-A- 5 393 785
- PROC. SOC. EXP. BIOL. MED., vol. 194, no. 1, 1990, pages 54-57, XP002021651 B.W. SNYDER ET AL.: "Danazol Suppression of Luteinizong Hormone in the rat: Evidence for mediation by both androgen and estrogen receptors"
- DATABASE PHARMAPROJECTS PJB Publications, Ltd. DIALOG Accession No. 0011501, 19 March 1993 "ICI-164384" XP002021705 & 79th AACR (New Orleans), 1988, Abs 104
- J. BONE MINERAL RES., vol. 8, no. suppl 1, 1993, page s157 XP002021652 J.M. CAIN ET AL.: "Combination of raloxifene and human parathyroid hormone 1-34 increase femur bone mass in young ovariectomized (OVX) rats"

## Description

The present invention relates to the fields of pharmacology and pharmaceutical chemistry, and provides uses for minimizing the bone loss effect induced by the administration of certain pharmaceutical agents, and pharmaceutical compositions therefor.

### Compounds of formula I

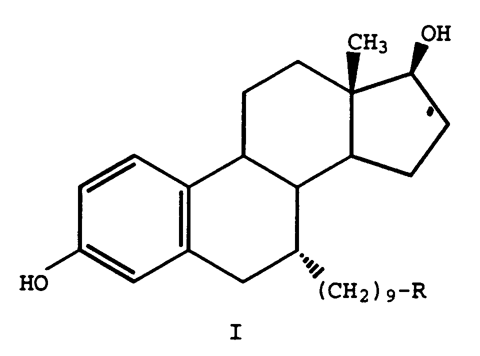

wherein R is -CH₂CON(CH₃)-n-CH₂CH₂CH₂CH₃ or -SO(CH₂)₃CF₂CF₃, or a pharmaceutically acceptable salt thereof are known in the art as ICI-164384 and ICI-182780, respectively (see, e.g., European Publication 138504; and U.S. Pat. No. 4,659,516). These steroidal compounds have been characterized as Type III antiestrogens by McDonald, D.P., et al., (Steroid Receptor and Anti-Hormones Symposia, New York Acad. Sci., Dallas, TX, Sept.
20-23, 1994), and have further been classified as "pure" antiestrogens which are devoid of agonist activity. As such, compounds of formula I bind to the estrogen receptor (ER) with high affinity, thus blocking the binding of estrogen and its metabolites. These compounds also appear to inhibit ER dimerization and facilitate cytoplasmic accumulation of ER leading to rapid degradation resulting in loss of immunodetectable protein. Accordingly, these compounds are understood to be promising therapeutic agents for the treatment of inter alia, breast cancer [see, e.g., Howell, A., Lancet, 345:29-30 (1995)] endometriosis, uterine fibroids, and other estrogen-induced maladies.

Unfortunately, compounds of formula I are devoid of estrogen agonist activity and, thus, induce a state similar to that seen in postmenopausal women. More particularly, these compounds are capable of inducing bone loss such as seen with osteoporosis. The induction of a postmenopausal state and, particularly, the induction of bone loss, is one of the most limiting side-effects of these compounds when administered for estrogen-induced maladies in mammals. It, therefore, would be of great value to be able to take advantage of the distinct antiestrogenic activity of formula I compounds while minimizing the negative side effects associated with the use of the compounds via the sequential or concurrent administration of another pharmaceutical agent.

The present invention, therefore, provides a use of minimizing the bone loss effect of a compound of formula I 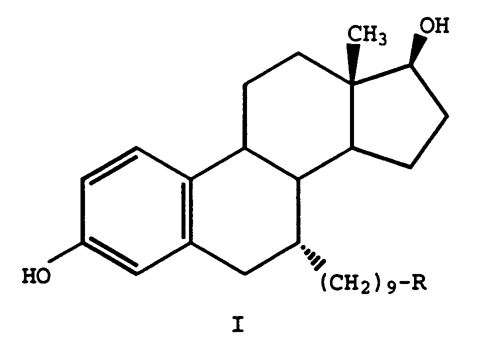 wherein R is -CH₂CON(CH₃)-n-CH₂CH₂CH₂CH₃ or -SO(CH₂)₃CF₂CF₃, or a pharmaceutically acceptable salt thereof, and wherein said formula I compound is to be administered to a mammal in need of treatment, comprising concurrently or sequentially administering to said mammal an effective amount of a compound of formula II 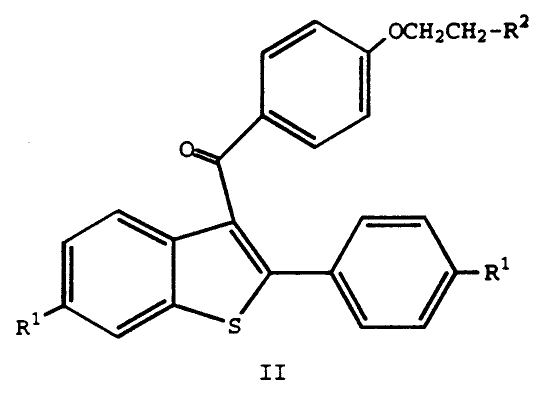 wherein
each R¹ is independently -H, -OH, -O(C₁-C₄ alkyl), -OCOC₆H₅, -OCO(C₁-C₆ alkyl), or -OSO₂(C₄-C₆ alkyl); and
R² is 1-piperidinyl, 1-pyrrolidinyl, methyl-1-pyrrolidinyl, dimethyl-1-pyrrolidinyl, 4-morpholino, dimethylamino, diethylamino, or 1-hexamethyleneimino;
   or a pharmaceutically acceptable salt thereof.

The present invention further provides pharmaceutical formulations comprising a formula I compound and a formula II compound, with or without a bone anabolic agent, and a formula I compound plus a bone anabolic agent, in combination with a pharmaceutically acceptable carrier, diluent, or excipient.

Compounds of formula I 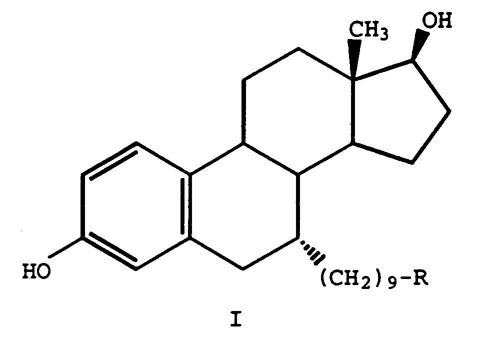 wherein R is -CH₂CON(CH₃)-n-CH₂CH₂CH₂CH₃ or -SO(CH₂)₃CF₂CH₃, or a pharmaceutically acceptable salt thereof, are well known in the art and are prepared as taught by Bowler, J., in U.S. Pat. No. 4,659,516, which is herein incorporated by reference. A preferred formula I compound is that in which R is -SO(CH₂)₃CF₂CH₃ (ICI-182780).

Similarly, compounds of formula II 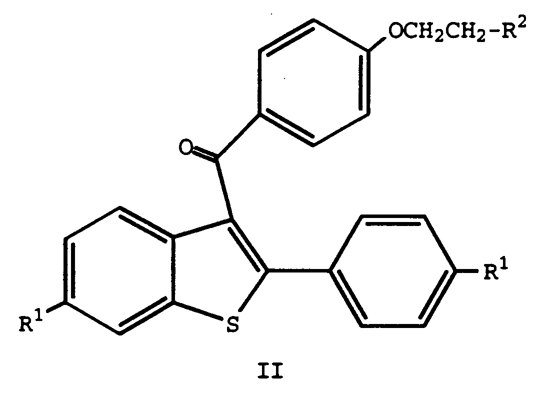 wherein
each R¹ is independently -H, -OH, -O(C₁-C₄ alkyl), -OCOC₆H₅, -OCO(C₁-C₆ alkyl), or -OSO₂(C₄-C₆ alkyl); and
R² is 1-piperidinyl, 1-pyrrolidinyl, methyl-1-pyrrolidinyl, dimethyl-1-pyrrolidinyl, 4-morpholino, dimethylamino, diethylamino, or 1-hexamethyleneimino;
   or a pharmaceutically acceptable salt thereof, are well known in the art and can be prepared according to established procedures, such as those detailed in U.S. Pat. Nos. 4,133,814; 4,418,068; and 4,380,635, each of which is incorporated by reference.

Compounds of formula II, particularly raloxifene, in which each R¹ is -OH and R² is 1-piperidinyl, has been classified by McDonald, supra, as a type II antiestrogen. Compounds of formula II also are recognized in the art as selective estrogen receptor modulators (SERMs), or tissue selective estrogen receptor agonists/antagonists. As such, the compounds of formula II are recognized as nuclear regulatory molecules. More particularly, raloxifene has been shown to bind to estrogen receptors and originally was demonstrated to have antiestrogenic activity because it blocked the ability of estrogen to activate uterine tissue and estrogen-dependent cancers. Indeed, raloxifene does block the action of estrogen in some cells but, in other cell types, it activates the same genes as estrogen activates and displays the same pharmacology. As a type II antiestrogen, SERM, raloxifene, and its analogs defined above as compounds of formula II, are tissue selective antiestrogens with mixed agonist-antagonist properties.

Although compounds of formulae I and II generally compete for and utilize the same receptors, the pharmacological outcome of administration of these distinct agents is not easily predicted, and is distinct to each.

Bone anabolic agents are those agents which are known in the art to build bone by increasing the production of bone matrix protein. Such anabolic agents include, for example, the various forms of parathyroid hormone (PTH) such as naturally occurring PTH (1-84), PTH (1-34), analogs thereof, and the like, which are prepared via well known procedures.

As used herein, "bone loss" means a reduction of bone mineral density of cancellous bone, which frequently is a side-effect of formula I compound administration to mammals, and the term "minimize", or a derivative thereof, contemplates partial or complete inhibition and/or repair of a compound of formula I-induced bone loss.

The uses of the present invention can be tailored to counter the bone loss effect induced by the administration of a formula I compound. For example, when administration of a formula I compound is first initiated, particularly as an acute treatment, it is preferred to coadminister a compound of formula II, especially the hydrochloride salt of raloxifene, to counteract the potential bone loss. When administration of a formula I compound will be for the treatment of a chronic malady (e.g., endometriosis; cancer adjuvant following surgery), a formula II compound, preferably raloxifene hydrochloride, and an anabolic bone agent, particularly PTH (1-84) or PTH (1-34), may be coadministered at the time treatment with a formula I compound is initiated, and throughout the course of therapy. However, if a formula I compound is administered for a chronic malady without the coadministration of a formula II compound, a bone anabolic agent may be coadministered with, or following, multiple courses of therapy with a formula I compound. The particular method of the present invention which would optimize the minimization of bone loss induced by the administration of a formula I compound is, therefore, dictated by the duration of a formula I compound's course of therapy, and when administration of a compound of formula II, and optionally a bone anabolic agent, is initiated relative to the commencement of therapy of a formula I compound. In essence, the attending physician is best suited to determine whether a formula II compound and optionnally a bone anabolic agent should be administered, and whether the administration of such agents should be concurrent or sequential to the administration of a formula I compound.

When administered sequentially, pharmaceutical formulations of compound of formulae I and II and bone anabolic agents are prepared by methods known by one of ordinary skill in the art.

When administered concurrently, formulae I and II compounds and bone anabolic agents may be prepared into pharmaceutical formulations via the above-mentioned known methods, and administered as separate entities. Alternatively, they may be combined to form a pharmaceutical composition of the present invention which comprises a compound of formula I, or a pharmaceutically acceptable salt thereof, a compound of formula II, or a pharmaceutically acceptable salt thereof, and, optionally, a bone anabolic agent, in combination with a pharmaceutically acceptable carrier, diluent, or excipient.

The present invention also provides pharmaceutical compositions comprising a compound of formula I, or a pharmaceutically acceptable salt thereof, and a bone anabolic agent, in combination with a pharmaceutically acceptable carrier diluent, or excipient.

Preferred compounds of formulae I and II and bone anabolic agents for pharmaceutical compositions of the present invention are the same as those preferred for the uses of the present invention.

Pharmaceutical compositions of the present invention can be prepared in unit dosage form for parenteral, transdermal, rectal, nasal, intravenous, or oral administration via conventional and well known techniques. Such compositions comprise active ingredient of each desired combinant will be mixed with a carrier, diluted by a carrier, or enclosed within a carrier which may be in the form of a capsule, sachet, paper, or other container. When the carrier serves as a diluent, it may be a solid, semisolid, or liquid material which acts as a vehicle, excipient, or medium for the active ingredient. Thus, the compositions of the present invention can be in the form of tablets, pills, powders, lozenges, sachets, soft and hard gelatin capsules, sterile injectable solutions, and sterile packaged powders. As used herein, the term "active ingredient" refers to the respective formula I compound, or a pharmaceutically acceptable salt thereof, a formula II compound, or a pharmaceutically acceptable salt thereof, and a bone anabolic agent, used in a pharmaceutical composition of the present invention.

Additionally, pharmaceutical agents of the present compositions are well suited for formulation as sustained release dosage forms and the like. The compositions can be so constructed that they release active ingredient only or preferably in a particular physiological location, preferably over a long period of time. The coatings envelope and protective matrices may be made, for example from polymeric substances or waxes.

More particularly, pharmaceutical compositions of the present invention which sequentially release, for example, an effective amount of a compound of formula I, followed by the release of an effective amount of a compound of formula II, and optionally a bone anabolic agent, may be constructed as an implant device. Such an implant device would consist of an outer, rapidly degradable polymer, such as a low molecular weight wax, impregnated with a compound of formula I. The inner cone of the implant would be made of a slowly degradable polymer, such as a high molecular weight wax, impregnated with a compound of formula II and optionally a bone anabolic agent.

Also included within the scope of the present invention are pharmaceutical compositions for transdermal delivery of the pharmaceutical agents used in the methods herein described. The preparation of such compositions are well known to one of ordinary skill in the art.

Some examples of suitable carriers, excipients, and diluents include lactose, dextrose, sucrose, sorbitol, mannitol, starches, gum acacia, calcium phosphate alginates, calcium salicate, microcrystalline cellulose, polyvinylpyrrolidone, cellulose, tragacanth, gelatin, syrup, methyl cellulose, methyl- and propylhydroxybenzoates, talc, magnesium stearate, water, and mineral oil. The compositions can additionally include lubricating agents, wetting agents, emulsifying and suspending agents, preserving agents, sweetening agents or flavoring agents. The compositions may be formulated so as to provide quick, sustained, or delayed release of the active ingredient(s) after administration to the patient by employing procedures well known in the art. For oral administration, a compound optionally including a second component compound, can be admixed with carriers and diluents molded into tablets or enclosed in gelatin capsules. The mixtures can alternatively be dissolved in liquids such as 10% aqueous glucose solution, isotonic saline or sterile water, and administered intravenously or by injection.

The compositions are preferably formulated in a unit dosage form, each dosage containing from 1 to 500 mg and, more frequently, from 5 to 300 mg of the active ingredient(s). The term "unit dosage form" refers to physically discreet units suitable as unitary dosages for human subjects, each unit containing a predetermined quantity of active ingredients calculated to produce the desired therapeutic effect, in association with the required pharmaceutically acceptable carrier. By "pharmaceutically acceptable", it is meant the carrier, diluent, or excipient must be acceptable with the other ingredients of the formulation and not deleterious to the recipient thereof.

The following formulation and composition examples are only illustrative and are not intended to limit the scope of the present invention.

### Formulations/Compositions

### Formulation 1: Gelatin capsules

Hard gelatin capsules are prepared using the following:

| Ingredient | Quantity (mg/capsule) |
|---|---|
| Formula I compound | 0.1 - 1000 |
| Starch, NF | 0 - 650 |
| Starch flowable powder | 0 - 650 |
| Silicone fluid 3.5 x 10⁻⁴m²/s (350 centistokes) | 0 - 15 |

### Formulation 2: Raloxifene capsule

| Ingredient | Quantity (mg/capsule) |
|---|---|
| Raloxifene HCl | 10 |
| Starch, NF | 103 |
| Starch flowable powder | 225.3 |
| Silicone fluid 3.5 x 10⁻⁴m²/s (350 centistokes) | 1.7 |

### Formulation 3: Raloxifene capsule

| Ingredient | Quantity (mg/capsule) |
|---|---|
| Raloxifene HCl | 50 |
| Starch, NF | 150 |
| Starch flowable powder | 397 |
| Silicone fluid 3.5 x 10⁻⁴m²/s (350 centistokes) | 3.0 |

The specific formulations above may be changed in compliance with the reasonable variations provided.

A tablet formulation is prepared using the ingredients below:

### Formulation 4: Tablet

| Ingredient | Quantity (mg/tablet) |
|---|---|
| Formula I compound | 2.5 - 1000 |
| Cellulose, microcrystalline | 200 - 650 |
| Silicon dioxide, fumed | 10 - 650 |
| Stearate acid | 5 - 15 |

The components are blended and compressed to form tablets.

Alternatively, tablets each containing 25 - 1000 mg of a formula I compound are made up as follows:

### Formulation 5: Tablet

| Ingredient | Quantity (mg/tablet) |
|---|---|
| Formula I compound | 25 - 1000 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The formula I compound, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°-60° C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 25 - 1000 mg of medicament per 5 ml dose are made as follows:

### Formulation 6: Suspension

| Ingredient | Quantity (mg/5 ml) |
|---|---|
| Formula I compound | 25 - 1000 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The medicament is passed through a No. 45 mesh U.S. sieve and mixed with the sodium carboxymethyl cellulose and syrup to form a smooth paste. The benzoic acid solution, flavor, and color are diluted with some of the water and added, with stirring. Sufficient water is then added to produce the required volume.

### Composition 1: Intravenous Solution

| Ingredient | Quantity |
|---|---|
| Formula I compound | 50 mg |
| Formula II compound | 50 mg |
| Isotonic saline | 1000 mL |

### Composition 2: Intravenous Solution

| Ingredient | Quantity |
|---|---|
| Formula I compound | 50 mg |
| PTH (1-84) or (1-34) | 0.1-1000 mg |
| Isotonic saline | 1000 mL |

### Formulation 3: Intravenous Solution

| Ingredient | Quantity |
|---|---|
| Formula I compound | 50 mg |
| Formula II compound | 50 mg |
| PTH (1-84) or (1-34) | 0.1-1000 mg |
| Isotonic saline | 1000 mL |

The particular dosage of a compound of formula II, particularly raloxifene, with or without the coadministration of a bone anabolic agent, particularly PTH (1-84) or (1-34), required to minimize the bone loss effect of a compound of formula I according to the present invention will depend upon the severity of the condition, the route of administration, and related factors which will be decided by the attending physician.

Generally, accepted and effective daily doses of a formula II compound will be from 0.1 mg to 1000 mg/day, and more typically from 50 mg to 250 mg/day. Such dosages will be administered to a mammal in need of treatment from once to about three times each day, or more often as needed to effectively treat the present indication. It, usually, is preferred to administer a formula II compound in the form of an acid addition salt, especially, via the oral route.

Preferred dosages, routes of administration, and frequency of administration of formula I compounds and bone anabolic agents are well established and known to those of ordinary skill in the art.

### Test Procedures

### Bone Loss I

It is well established in the literature that the ovariectomized rat model is a reasonable model for studying bone loss, particularly osteopenia observed in estrogen-deficient states such as postmenopausal osteoporosis [see, e.g., Wronski, T.J., et al., Cells Mater. Supp, 1:69-74 (1991)]. In this model, compounds of formula I, particularly a formula I compound in which R is -CH₂CON(CH₃)-n-CH₂CH₂CH₂CH₃, did not reduce bone loss when administered at a dose from 0.03 mg to 10 mg/kg P.O. Because the bone loss observed in this model is reflective of the bone loss induced by the administration of a formula I compound, administration of a formula II compound, with or without a bone anabolic agent demonstrates the efficacy of the administered compounds for minimizing the bone loss induced by a formula I compound.

### Bone Loss II

Compounds of formula I have been shown to induce bone loss in normal female rats [see, e.g., Gallagher, A., Endocrinology, 133(6):2787-2791 (1993)]. Under the conditions of this experiment, when bone loss is occurring, the coadministration of a formula II compound, with or without a bone anabolic agent, minimizes this bone loss.

### Bone Loss III

Using the ovarectomized model described in Bone Loss I, an alternating schedule of administering a compound of formula I, followed by the administration of a compound of formula II, with or without a bone anabolic agent demonstrates the ability of compounds of formula II and bone anabolic agents to minimize the bone loss induced by the administration of formula I compounds. In this model, rats treated only with a compound of formula I is used as a control. The duration of this study may run up to six months or beyond.

### Bone Loss IV

In normal female rats, bone loss can be induced by a compound of formula I. A cyclic regimen of therapy of a compound of formula I, alternating with a compound of formula II, minimizes the loss of bone induced by a formula I compound when compared to a control group which has been continuously treated with a compound of formula II. Specifically, three groups of animals are chosen. The first group would be a no treatment control group. The second group would receive a S.C. dose of a formula I compound in an oily suspension every 21 days for six months. The third group would receive an alternating schedule of therapy consisting of a compound of formula I followed after 21 days with a daily dose of a formula II compound for 14 days. This protocol would be repeated for six months. At the end of the experiment, the bone mineral density is determined by DEXA (Dual Energy X-ray Analysis), demonstrating the bone loss efficacy of the coadministered compound of formula II.

### Bone Loss V

Twenty to fifty women with diagnosed estrogen-dependent breast cancer are selected for the study. These women may be pre- or post-menopausal, have a WHO performance status of less than two, and have a life expectancy of greater than one year. Further, these women may or may not have had previous surgical intervention and should not have been treated with tamoxifene or other anti-cancer chemotherapeutic agents.

These patients are initially evaluated for their cancer status by techniques known in the art, (see, e.g., Howell, A., supra, and references cited therein). In addition, the patients are evaluated for bone status by monitoring bone mineral density by DEXA, urinary calcium, creatinine, hydroxyproline, and pyridinoline crosslinks.

Patients are treated for cancer by monthly injection of an oily suspension of 100-250 mg of a formula I compound. During monthly visits to the attending physician, bone mineral density and the above bone markers are evaluated. Either immediately, or when bone loss reaches about 10% to 20% of the base line measurement, patients additionally receive 50-250 mg of raloxifene, P.O., for the remainder of the trial period. The duration of the present trial is one year.

This trial demonstrates the minimization of bone loss via administration of a formula I compound while not affecting the cancer efficacy of a formula I compound.

Alternatively, a bone anabolic agent, particularly PTH (1-84) or (1-34), can be administered in addition to the administration of a formula II compound.

## Claims

1. The use of a compound of formula II 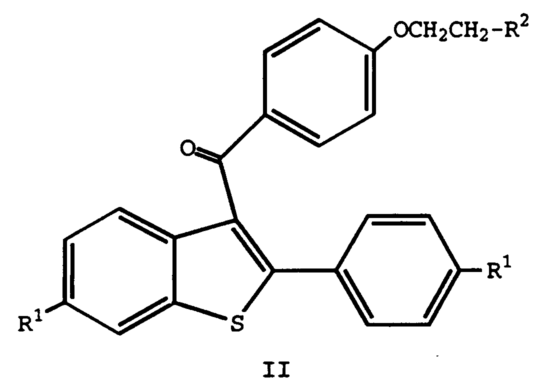 wherein
each R¹ is independently -H, -OH, -O(C₁-C₄ alkyl), -OCOC₆H₅, -OCO(C₁-C₆ alkyl), or -OSO₂(C₄-C₆ alkyl); and
R² is 1-piperidinyl, 1-pyrrolidinyl, methyl-1-pyrrolidinyl, dimethyl-1-pyrrolidinyl, 4-morpholino, dimethylamino, diethylamino, or 1-hexamethyleneimino;
or a pharmaceutically acceptable salt thereof, in the preparation of a medicament for minimizing the bone loss effects of a compound of formula I 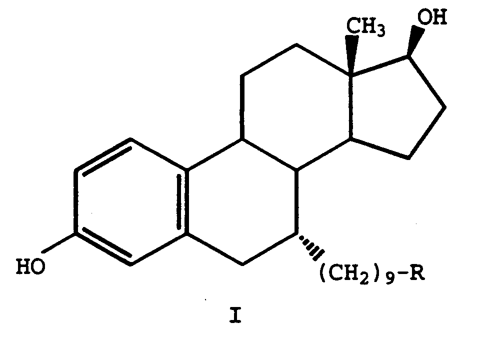 wherein R is -CH₂CON(CH₃)-n-CH₂CH₂CH₂CH₃ or -SO(CH₂)₃CF₂CF₃, or a pharmaceutically acceptable salt thereof.

2. A use according to Claim 1 wherein R of said formula I compound is -SO(CH₂)₃CF₂CF₃, or a pharmaceutical acceptable salt thereof, each R¹ of said formula II compound is -OH, and R² of said formula II compound is 1-piperidinyl, or a pharmaceutically acceptable salt thereof.

3. A use according to Claim 1 wherein R of said formula I compound is -CH₂CON(CH₃)-n-CH₂CH₂CH₂CH₃, or a pharmaceutically acceptable salt thereof, each R¹ of a formula II compound is -OH, and R² of said formula II compound is 1-piperidinyl, or a pharmaceutically acceptable salt thereof.

4. A pharmaceutical composition comprising a compound of formula I 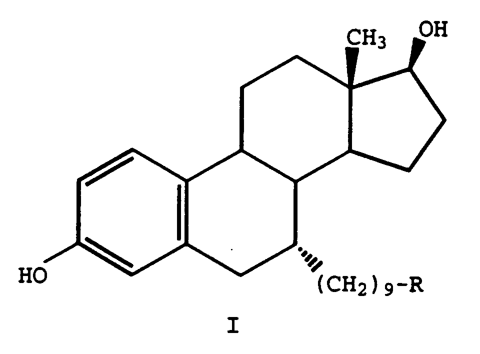 wherein R is -CH₂CON(CH₃)-n-CH₂CH₂CH₂CH₃ or -SO(CH₂)₃CF₂CF₃, or a pharmaceutically acceptable salt thereof, a compound of formula II 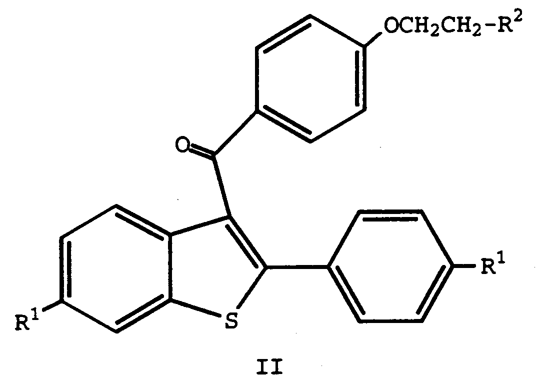 wherein
each R¹ is independently -H, -OH, -O(C₁-C₄ alkyl), -OCOC₆H₅, -OCO(C₁-C₆ alkyl), or -OSO₂(C₄-C₆ alkyl); and
R² is 1-piperidinyl, 1-pyrrolidinyl, methyl-1-pyrrolidinyl, dimethyl-1-pyrrolidinyl, 4-morpholino, dimethylamino, diethylamino, or 1-hexamethyleneimino;
or a pharmaceutically acceptable salt thereof; and optionally, a bone anabolic agent, in combination with a pharmaceutically acceptable carrier, diluent, or excipient.

5. A pharmaceutical composition according to Claim 4 wherein R of said formula I compound is -SO(CH₂)₃CF₂CF₃, or a pharmaceutically acceptable salt thereof, each R¹ of said formula II compound is -OH, and R² of said formula II compound is 1-piperidinyl, or a pharmaceutically acceptable salt thereof.

6. A pharmaceutical composition according to Claim 4 wherein R of said formula I compound is -CH₂CON(CH₃)-n-CH₂CH₂CH₂CH₃, or a pharmaceutically acceptable salt thereof, each R¹ of said formula II compound is -OH, and R² of said formula II compound is 1-piperidinyl,
or a pharmaceutically acceptable salt thereof.

## Patentansprüche

1. Verwendung einer Verbindung der Formel II 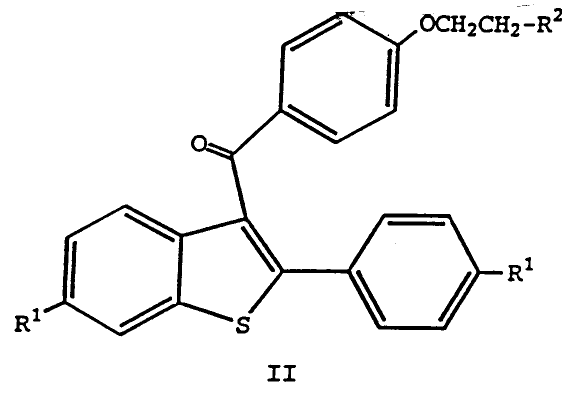 worin
jedes R¹ unabhängig für -H, -OH, -O(C₁-C₄ Alkyl), -OCOC₆H₅, -OCO(C₁-C₆ Alkyl) oder -OSO₂(C₄-C₆ Alkyl) steht und
R² für 1-Piperidinyl, 1-Pyrrolidinyl, Methyl-1-pyrrolidinyl, Dimethyl-1-pyrrolidinyl, 4-Morpholino, Dimethylamino, Diethylamino oder 1-Hexamethylenimino steht oder eines pharmazeutisch annehmbaren Salzes hiervon, zur Herstellung eines Arzneimittels zur Minimierung der Knochenverlusteffekte durch eine Verbindung der Formel I 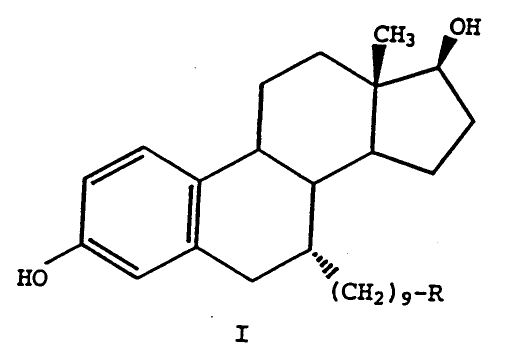 worin R für -CH₂CON(CH₃)-n-CH₂CH₂CH₂CH₃ oder -SO(CH₂)₃CF₂CF₃ steht, oder durch ein pharmazeutisch annehmbares Salz hiervon.

2. Verwendung nach Anspruch 1, worin R bei der Verbindung der Formel I für -SO(CH₂)₃CF₂CF₃ steht, oder diese Verbindung ein pharmazeutisch annehmbares Salz ist, wobei jedes R¹ bei der Verbindung der Formel II für -OH steht und R² bei der Verbindung der Formel II für 1-Piperidinyl steht, oder diese Verbindung ein pharmazeutisch annehmbares Salz ist.

3. Verwendung nach Anspruch 1, worin R bei der Verbindung der Formel I für -CH₂CON(CH₃)-n-CH₂CH₂CH₂CH₃ steht, oder diese Verbindung ein pharmazeutisch annehmbares Salz ist, wobei jedes R¹ bei der Verbindung der Formel II für -OH steht und R² bei der Verbindung der Formel II für 1-Piperidinyl steht, oder diese Verbindung ein pharmazeutisch annehmbares Salz ist.

4. Pharmazeutische Zusammensetzung, die umfaßt eine Verbindung der Formel I, 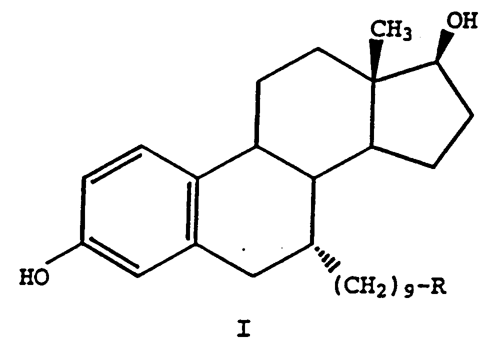 worin R für -CH₂CON(CH₃)-n-CH₂CH₂CH₂CH₃ oder -SO(CH₂)₃CF₂CF₃ steht, oder ein pharmazeutisch annehmbares Salz hiervon, eine Verbindung der Formel II 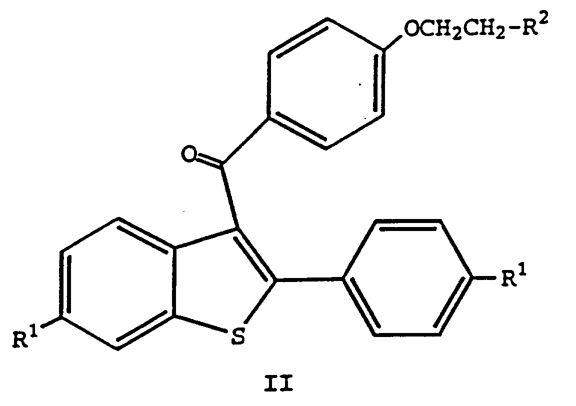 worin
jedes R¹ unabhängig für -H, -OH, -O-(C₁-C₄ Alkyl), -OCOC₆H₅, -OCO(C₁-C₆ Alkyl) oder -OSO₂(C₄-C₆ Alkyl) steht und
R² für 1-Piperidinyl, 1-Pyrrolidinyl, Methyl-1-pyrrolidinyl, Dimethyl-1-pyrrolidinyl, 4-Morpholino, Dimethylamino, Diethylamino oder 1-Hexamethylenimino steht oder ein pharmazeutisch annehmbares Salz hiervon, und wahlweise ein anaboles Knochenmittel in Kombination mit einem pharmazeutisch annehmbaren Träger, Verdünnungsmittel oder Hilfsstoff.

5. Pharmazeutische Zusammensetzung nach Anspruch 4, worin R bei der Verbindung der Formel I für -SO(CH₂)₃CF₂CF₃ steht, oder diese Verbindung ein pharmazeutisch annehmbares Salz ist, wobei jedes R¹ bei der Verbindung der Formel II für -OH steht und R² bei der Verbindung der Formel II für 1-Piperidinyl steht, oder diese Verbindung ein pharmazeutisch annehmbares Salz ist.

6. Pharmazeutische Zusammensetzung nach Anspruch 4, worin R bei der Verbindung der Formel I für -CH₂CON(CH₃)-n-CH₂CH₂CH₂CH₃ steht, oder diese Verbindung ein pharmazeutisch annehmbares Salz ist, wobei jedes R¹ bei der Verbindung der Formel II für -OH steht und R² bei der Verbindung der Formel II für 1-Piperidinyl steht, oder diese Verbindung ein pharmazeutisch annehmbares Salz ist.

## Revendications

1. Utilisation d'un composé de formule II 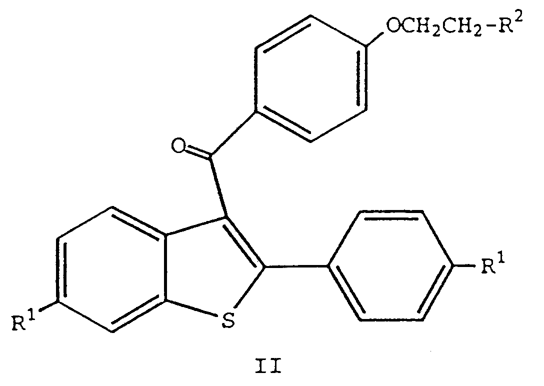 dans laquelle
chaque R¹ représente indépendamment -H,-OH, -O(alkyle en C₁-C₄), -OCOC₆H₅, -OCO(alkyle en C₁-C₆) ou -OSO₂(alkyle en C₄-C₆); et
R² représente un 1-pipéridinyle, un 1-pyrrolidinyle, un méthyl-1-pyrrolidinyle, un diméthyl-1-pyrrolidinyle, un 4-morpholino, un diméthylamino, un diéthylamino ou un 1-hexaméthylèneimino; ou un sel pharmaceutiquement acceptable de ceux-ci, dans la préparation d'un médicament visant à minimiser les effets de perte osseuse d'un composé de formule I 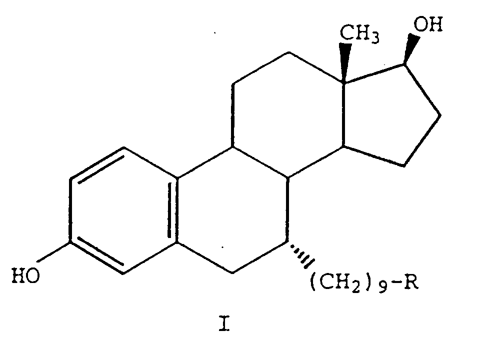 dans laquelle R représente -CH₂CON(CH₃)-n-CH₂CH₂CH₂CH₃ ou -SO(CH₂)₃CF₂CF₃ ou un sel pharmaceutiquement acceptable de ceux-ci.

2. Utilisation selon la revendication 1, dans laquelle, dans ledit composé de formule I, R représente -SO(CH₂)₃CF₂CF₃ ou un sel pharmaceutiquement acceptable de celui-ci, dans ledit composé de formule II, chaque R¹ représente -OH, et dans ledit composé de formule II, R² représente le 1-pipéridinyle ou un sel pharmaceutiquement acceptable de celui-ci.

3. Utilisation selon la revendication 1, dans laquelle, dans ledit composé de formule I, R représente -CH₂CON(CH₃)-n-CH₂CH₂CH₂CH₃ ou un sel pharmaceutiquement acceptable de celui-ci, dans ledit composé de formule II, chaque R¹ représente -OH, et dans ledit composé de formule II, R² représente le 1-pipéridinyle ou un sel pharmaceutiquement acceptable de celui-ci

4. Composition pharmaceutique comprenant un composé de formule I 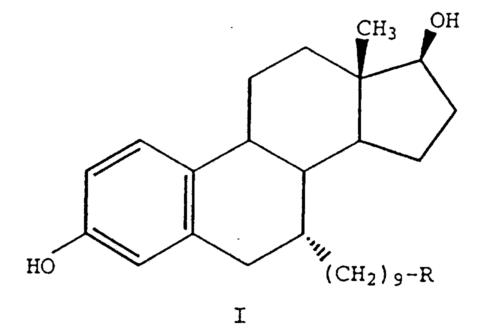 dans laquelle R représente -CH₂CON(CH₃)-n-CH₂CH₂CH₂CH₃ ou -SO(CH₂)₃CF₂CF₃ ou un sel pharmaceutiquement acceptable de ceux-ci, un composé de formule II 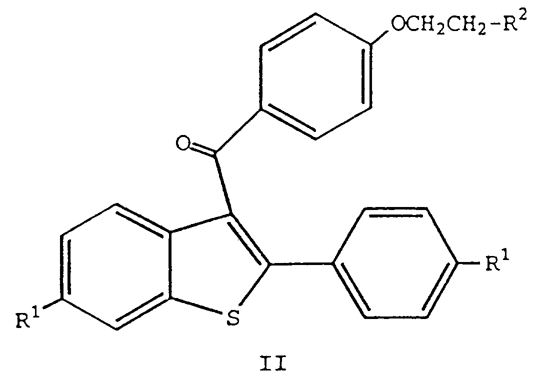 dans laquelle
chaque R¹ représente indépendamment -H, -OH, -O(alkyle en C₁-C₄), -OCOC₆H₅, -OCO(alkyle en C₁-C₆) ou ―OSO₂(alkyle en C₄-C₆); et
R² représente un 1-pipéridinyle, un 1-pyrrolidinyle, un méthyl-1-pyrrolidinyle, un diméthyl-1-pyrrolidinyle, un 4-morpholino, un diméthylamino, un diéthylamino ou un 1-hexaméthylèneimino; ou un sel pharmaceutiquement acceptable de ceux-ci; et
facultativement, un anabolisant de l'os, en combinaison avec un porteur, un diluent ou un excipient pharmaceutiquement acceptables.

5. Composition pharmaceutique selon la revendication 4, dans laquelle, dans ledit composé de formule I, R représente -SO(CH₂)₃CF₂CF₃ ou un sel pharmaceutiquement acceptable de celui-ci, dans ledit composé de formule II, chaque R¹ représente -OH, et dans ledit composé de formule II, R² représente le 1-pipéridinyle ou un sel pharmaceutiquement acceptable de celui-ci.

6. Composition pharmaceutique selon la revendication 4, dans laquelle, dans ledit composé de formule I, R représente -CH₂CON(CH₃)-n-CH₂CH₂CH₂CH₃ ou un sel pharmaceutiquement acceptable de celui-ci, dans ledit composé de formule II, chaque R¹ représente -OH, et dans ledit composé de formule II, R² représente le 1-pipéridinyle ou un sel pharmaceutiquement acceptable de celui-ci.
